# EUROPEAN PATENT APPLICATION

(11) **EP 3 069 677 A1**
(43) Date of publication of application: **21.09.2016**
(21) Application number: 16160578.7
(22) Date of filing: 16.03.2016
(51) Int. Cl.: A61B 18/02, A61F 7/00

(54) **HANDPIECE ASSEMBLY FOR AN APPARATUS USED IN COSMETIC TREATMENT**

(30) Priority: 17.03.2015 FI 20155181
(71) Applicant: Cryotech Nordic Oü, 76903 Tallinn (EE)
(72) Inventor: Martins, Jean-Patrick, 76903 Tallinn (EE); Simonen, Ari, 01620 Vantaa (FI); Hirvonen, Vesa, 02720 Espoo (FI); Eklund, Jan, 01620 Vantaa (FI)
(74) Representative: IPR Partners Ltd

(57) **Abstract**

A handpiece assembly 100 for an apparatus used in non-invasive cosmetic treatment, in particular, in an apparatus for non-invasive fat reduction by means of cold-induced lipolysis of fat cell, known as cryolipolysis, is provided. Said handpiece assembly 100 comprises a handle 10, provided with a control terminal 14 equipped with a user interface, said handle 10 being connectable to the treatment unit of the apparatus for cosmetic treatment by a number of fluidic and/or communication lines; a treatment head 50 configured as a cup-shaped body applicator 20 disposed between at least two cooling units 30, and an adapter 40 configured to connect the handle 10 and the treatment head 50, wherein the connection between the handle 10 and the treatment head 50 mediated by the adapter 40 is releasable.

## Description

### FIELD OF THE INVENTION

The present invention generally relates to cosmetic treatment devices and systems for non-invasive reduction of fat deposits by controlled cooling of adipose tissue; more particularly the invention concerns an improved body applicator device for aforesaid equipment.

### BACKGROUND

In recent years a number of newly designed technologies for non-invasive body contouring and reshaping have become available to the consumer. These include reducing body fat deposits by means of high-intensity focused ultrasound, radiofrequency, low-level laser, acoustic waves and controlled cooling of adipose tissue to sub-zero temperatures (Celsius). The latter technique, also known as cryolipolysis, has proved to be one of the safest and most effective procedures for non-invasive body fat removal. Technique of cryolipolysis exploits the premise that adipocytes (fat cells) are more susceptible to low temperatures than other skin cells. Precise application of sub-zero temperatures onto dedicated areas triggers the process of fat cells' apoptosis (cell death), by which cells self-destruct without incurring damage to surrounding tissues. Exposing adipose tissue to low temperatures for prolonged periods of time (typically 30-60 min) causes fat cell membrane structure to collapse, upon which the lipids contained within the cell are released into lymphatic system and subsequently either converted by liver into blood sugar and/or eliminated via natural metabolic pathways. Since once destroyed adipocytes do not regenerate the results achieved by means of cryolipolysis are stable.

Devices for cryolipolysis generally employ e.g. vacuum pump connected cup-shaped applicators for creating vacuum suction when applied onto the treated area. Each device may include a number of vacuum applicators, generally varying from one to four. Vacuum temporarily decreases blood flow to the treated area thus immobilizing the fat tissue and isolating the treated area from surrounding tissue. Skin and subcutaneous tissue thus drawn into the cup-shaped vacuum applicator under moderate vacuum and the selected temperature is modulated by thermoelectric elements and controlled by sensors that monitor the heat flux out of the tissue.

In the United States patent application publication No. US 2010/0280582 a system is disclosed for removing heat from subcutaneous lipid-rich cells. The system comprises a treatment unit connected by a number of fluidic lines to a treatment head device including a vacuum applicator disposed between two Peltier-type thermoelectric elements. A handpiece for cryolipolysis device, said handpiece comprising a vacuum suction housing and two Peltier effect-based cooling plates is also known from the Korean patent application No. 2014-0133785.

Known prior art further includes a device for cryolipolysis trademarked as CTN Monolith Quattro Cryolipo, comprising four independently controlled treatment heads for controlled cooling the treated area under moderate vacuum, and additionally configured to irradiate the treated area by a low-level laser in order to protect the skin surface during the cooling period.

Above mentioned devices and systems are constrained with common technical problems. At first, provision of known devices for cryolipolysis is such that each body applicator/treatment head device is realized as a structure permanently or at least fixedly connected to a number of conduits, which mediate supply of coolant to the heat exchangers, supply of vacuum to the vacuum cup applicator etc., said conduits being in turn connected to an actual treatment unit and/or a vacuum pump. Replacement of a single treatment head therefore requires detaching the complementary conduit(s) from the treatment unit and replacing the whole body applicator/treatment head - conduit containing assembly. Such a procedure is laborious and time-consuming. Moreover, production costs for complex replacement parts, comprising e.g. a fluidic conduit fixedly associated with a treatment head, are high, which affects also maintenance expenses and market pricing for an actual treatment.

Another constraint concerns incapability of known cryolipolysis devices to monitor tissue temperature within the treated area. Although the temperature applied during typical treatment are low (-2° C to - 3° C), each patient possesses individual perception threshold for cold. In order to avoid tissue damages monitoring of tissue temperature during cryolipolysis treatment is desired.

Other drawbacks of some existing devices for cryolipolysis include insufficient efficiency of the cooling units and/or heat exchangers utilized therewithin, originated from low heat transfer rates with regards to small footprint area, which results in excessive energy consumption by these devices.

### SUMMARY OF THE INVENTION

An objective of the present invention is to obviate one or more problems arising from the limitations and disadvantages of the related art. The objective is achieved by various embodiments of a handhold body applicator assembly for an apparatus used in non-invasive cosmetic treatment, such as cryolipolysis.

Thereby, in one aspect of the invention a handhold body applicator assembly, further referred to as a handpiece assembly, is provided for an apparatus used in non-invasive cosmetic treatment, said handpiece assembly comprising an electronically controlled handle provided with a control terminal equipped with a user interface, said handle being connectable to the treatment unit of the apparatus for cosmetic treatment by a number of fluidic lines and/or signal communication lines; a treatment head comprising a cup-shaped body applicator and at least two cooling units, wherein the cup-shaped body applicator is disposed between said at least two cooling units; and an adapter implemented as a separate, replaceable component, configured to connect the electronically controlled handle and the treatment head while said handle and the related apparatus for cosmetic treatment being maintained in a functional switch-on state, wherein the connection between the handle and the treatment head mediated by the adapter is releasable. The handpiece assembly is preferably configured for the apparatus used in non-invasive fat reduction by means of cold-induced lipolysis of fat cells, also known as cryolipolysis.

The adapter of the handpiece assembly is thus configured to mediate detachment of the treatment head from and reconnection thereof to the electronically controlled handle, while said handle and the related apparatus for cosmetic treatment being maintained in a functional switch-on state.

The adapter of the handpiece assembly is configured to communicate electric signals between each cooling unit and the handle. In some embodiment the adapter-mediated connection between the handle and the treatment head is electro-mechanical and implemented by means of at least one plug-in connector. In some other embodiment said connection is magnetic and/or mediated by negative pressure (vacuum).

In some embodiments the cup-shaped body applicator of the handpiece assembly is a vacuum cup applicator and comprises a cup-shaped frame element configured to preserve constant shape while vacuum is drawn into the interior cavity thereof and an edge element configured to provide tight fitting onto the treated area.

In one preferred embodiment each cooling unit of the handpiece assembly is configured as a thermoelectric cooling unit and comprises at least one thermoelectric cooler element and at least one heat transfer element configured as a flat planar heat pipe consisting of a hermetically sealed vessel provided with a closed-loop capillary recirculation system with a working fluid and having a hot side in contact with said at least one thermoelectric cooler element and a cold side in contact with an external heat sink or ambient air. In some supplementary embodiment working liquid is acetone.

In another preferred embodiment the handpiece assembly further comprises at least one thermal camera sensor configured to monitor temperature of subcutaneous tissue within the treated area, said camera sensor being incorporated into the cup-shaped body applicator. In some other embodiment said thermal camera is incorporated into the adapter.

In some embodiments the handpiece assembly further comprises at least one light source in the form of a low-level laser LED, said light source being incorporated into the cup-shaped body applicator. In some other embodiments said light source is incorporated into the adapter.

In another aspect an apparatus for cosmetic treatment is provided, comprising a treatment unit and a number of the handpiece assemblies implemented in accordance with any embodiment disclosed hereby, wherein each handpiece assembly is connected to the treatment unit by a number of fluidic and/or communication lines, including, but not limited with a coolant fluidic line, a vacuum line and an electrical signal communication line, while the apparatus being maintained in a functional switch-on state.

In one preferred embodiment each individual fluidic- and signal communication line coupled to the handpiece assembly and comprised within the apparatus for cosmetic treatment is configured detachable, replaceable and reconnectable with regard to any connection port localized within the treatment unit, and each individual handpiece assembly is further configured detachable, replaceable and reconnectable with regard to any fluidic- and signal communication line coupled to the treatment unit, while the apparatus being maintained in a functional switch-on state.

In some embodiment the apparatus for cosmetic treatment is further configured to interrupt the at least coolant circulation, electric current supply and vacuum supply operational functions with regard to each individual handpiece assembly upon detaching of said handpiece assembly from the corresponding fluidic- and/or signal communication line or upon detaching of said corresponding fluidic- and/or signal communication line, coupled to the handpiece assembly, from the treatment unit of said apparatus; and to resume said operational functions upon restoration of physical connection between these components.

The utility of the present invention arises from a variety of reasons depending on each particular embodiment thereof. At first, the handpiece assembly disclosed hereby allows for changing and/or replacing a treatment head, provided in present disclosure as a portion having a cup-shaped body applicator disposed between cooling elements, without replacing a complex structure comprising a handhold body applicator fixedly connected to a fluidic conduit. Simple "one-click" connection between the cup-shaped applicator and an electronically controlled handle makes possible utilizing a wide variety of cup-shaped applicators having different size and geometry. Such an arrangement allows significantly reducing a number of "spare parts" required for each apparatus for cosmetic treatment, such as fluidic conduits and electronically controlled handhold devices, thus decreasing production costs, accordingly.

Further, the invention enables real-time monitoring of subcutaneous tissue temperature within the treated area while conducting a cosmetic treatment. Such an arrangement guarantees that the patient would not suffer skin and subcutaneous tissue damages caused by excessive cooling.

The invention further provides for compact and light-weight cooling units having high performance efficiency. Cooling units provided within the handpiece assembly disclosed hereby utilize flat heat pipe technology instead of common water circulation channels' containing heat exchangers. Continuously operated sealed heat pipe elements possess high reliability in thermal management systems.

The term "treated area" refers in present disclosure to a predetermined area on a patients' body, onto which a body applicator, configured as a vacuum cup applicator, is applied so that skin and subcutaneous tissue within said area is drawn into an interior cavity of said vacuum cup applicator by means of vacuum suction. The term "target area" is largely utilized as a synonym to the term "treated area". The term "body applicator" is utilized hereby with the reference to a patients' body.

The term "subcutaneous tissue" indicates in present disclosure tissue lying beneath the dermis and includes subcutaneous fat, or adipose tissue, which primarily is composed of lipid-rich cells, or adipocytes

The expression "a number of" refers herein to any positive integer starting from one (1), e.g. to one, two, or three.

The term "element" may herein refer also to a multi-part element with multiple functionally and optionally also physically connected elements in addition to single-part or integrated elements.

Different embodiments of the present invention will become apparent by consideration of the detailed description and accompanying drawings.

### BRIEF DESCRIPTION OF THE DRAWINGS

Fig. 1 is a perspective view of a handpiece assembly in accordance with one aspect of the invention; the treatment head and the handle components are detached.
Figs. 2A and 2B illustrate the assembly of Fig. 1; the treatment head and the handle components are partly assembled (Fig. 2A) and fully assembled (Fig. 2B).
Fig. 3A shows a partially exploded view and Figs. 3B and 3C show an exploded view of the assembly shown in Fig. 1.
Fig. 4 shows an exploded detailed view of a cooling unit and of a vacuum cup applicator provided within the assembly shown in Fig. 1.
Fig. 5 shows an exploded view of a treatment head adapter component provided within the assembly shown in Fig. 1.
Fig. 6 shows an exploded view of the handle component provided within the assembly shown in Fig. 1.
Figs 7A and 7B show a bottom view of the handle and the treatment head adapter components provided within the assembly shown in Fig. 1; solid (Fig. 7A) and transparent (Fig. 7B).
Fig. 7C shows a top view (solid) of the handle and the treatment head adapter components provided within the assembly shown in Fig. 1.
Fig. 8 shows an apparatus for the cosmetic treatment in accordance with some embodiment.

### DETAILED DESCRIPTION OF THE EMBODIMENTS

Detailed embodiments of the present invention are disclosed herein with the reference to accompanying drawings. The same reference characters are used throughout the drawings to refer to same members. Following citations are used for the members:
100 - a handpiece assembly;
10 - a handle;
11a, b, c - casing components;
12 - a protection plate (handle);
13 - a connector;
14 - a control terminal/user interface;
15 - fittings (handle);
16 - a fluidic line connector/adjusting control device;
17 - an aperture (handle);
20 - a vacuum cup applicator;
21 - a frame (vacuum cup applicator);
22 - an edge (vacuum cup applicator);
23 - a side aperture (vacuum cup applicator);
30 - a cooling unit;
31 - an outer cover;
32 - a heat transfer element;
33 - a thermoelectric cooler;
34 - an inner cover;
35 - a first cold plate;
36 - a second cold plate;
40 - an adapter for the treatment head;
41 - a vacuum cup connecting part;
42 - a protection plate (treatment head adapter);
43 - a connector (treatment head adapter);
44 - fittings (treatment head adapter);
45 - an angle fitting (treatment head adapter);
50 - a treatment head;
111 - a fluidic line;
200 - a treatment unit;
221 - user interface for the treatment unit;
500 - an apparatus for cosmetic treatment.

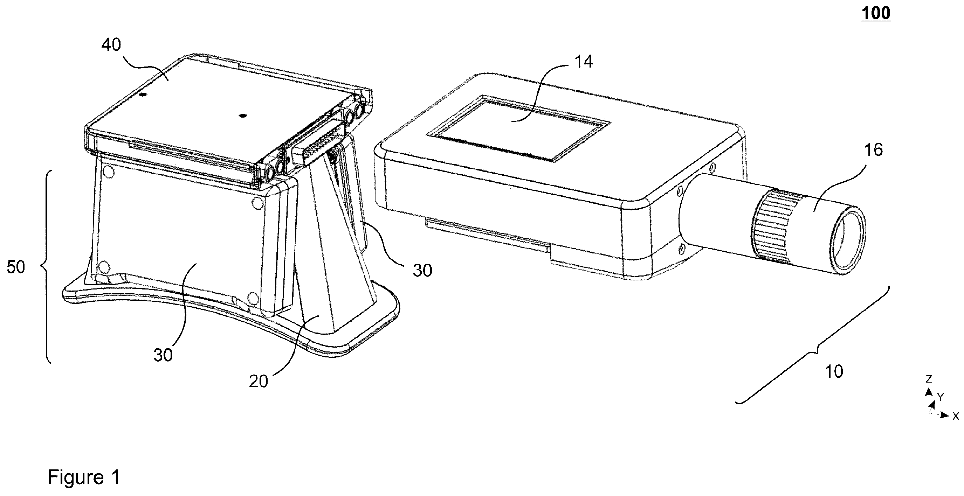

Fig. 1 illustrates at 100 the concept underlying various embodiments of a handpiece assembly of the present invention. Said handpiece assembly 100 is suitable for use with an apparatus used in cosmetic treatment, in particular, in the apparatus for non-invasive fat reduction by means of cold-induced fat cells' lipolysis, also referred to as cryolipolysis.

Such an apparatus integrates means for cooling adipose tissue to sub-zero Celsius temperatures with means for applying vacuum suction onto a treated area. The apparatus is preferably configured to affect the treated area by a low-level laser in order to protect patients' skin during treatment. Mentioned apparatus generally conforms to known technologies and comprises a treatment unit provided with a central user interface and a number of handhold body applicators configured for simultaneous work and connected with the treatment unit by a complementary number of fluidic lines, wherein each body applicator is implemented as a handpiece assembly 100 in accordance to the preferred embodiments disclosed hereby. The apparatus further includes at least one a vacuum pump and/or other auxiliary appliances. Preferably the apparatus is configured such that each handpiece assembly is connectable to a separate vacuum pump.

To cope with the potential problems disclosed in the background section the handpiece assembly 100 is provided. The assembly 100 according to one aspect of the invention constitutes a plug-in arrangement which enables replacement of a treatment head while fluidic line(s) remain connected to the treatment unit.

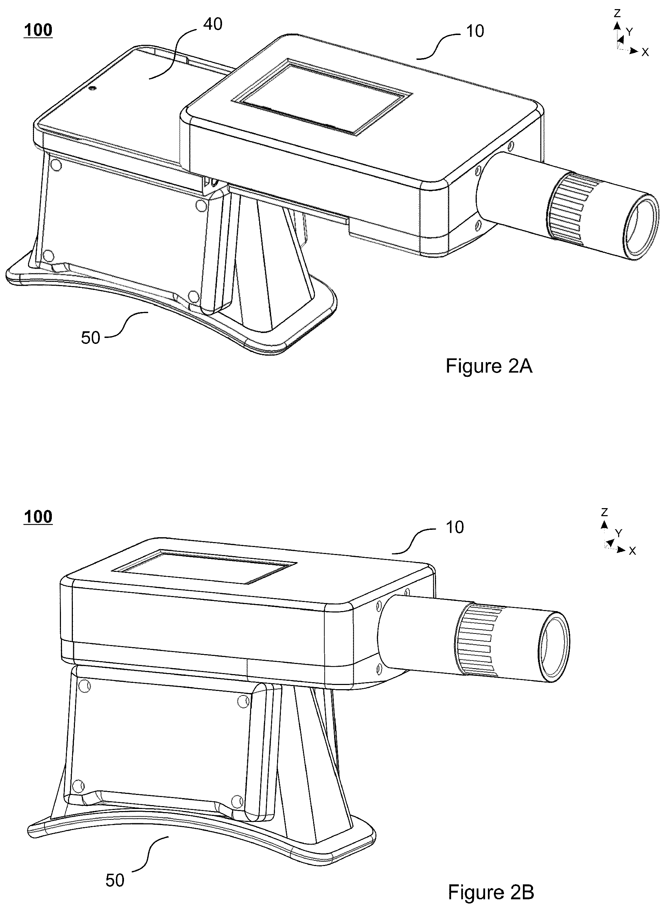

The assembly 100 shown in Fig. 1 thus comprises a treatment head 50 configured for cooling the treated area while applying vacuum suction thereto and an electronically controlled handle 10 equipped with individually adjustable vacuum power, temperature and time settings. The treatment head 50 comprises a cup-shaped body applicator 20 configured as a vacuum cup applicator and disposed between two cooling units 30. The handle 10 is provided with at least one electronic control terminal 14, preferably equipped with a user interface. The handle is connectable to fluidic- and/or communication line(s) (not shown) via a connector/adjusting device 16. Fig. 1 illustrates the assembly 100 having the treatment head 50 disconnected from the handle 10. Fig. 2A shows a process of joining the components 10 and 50 to each other, whereas Fig. 2B shows the assembly 100 fully connected.

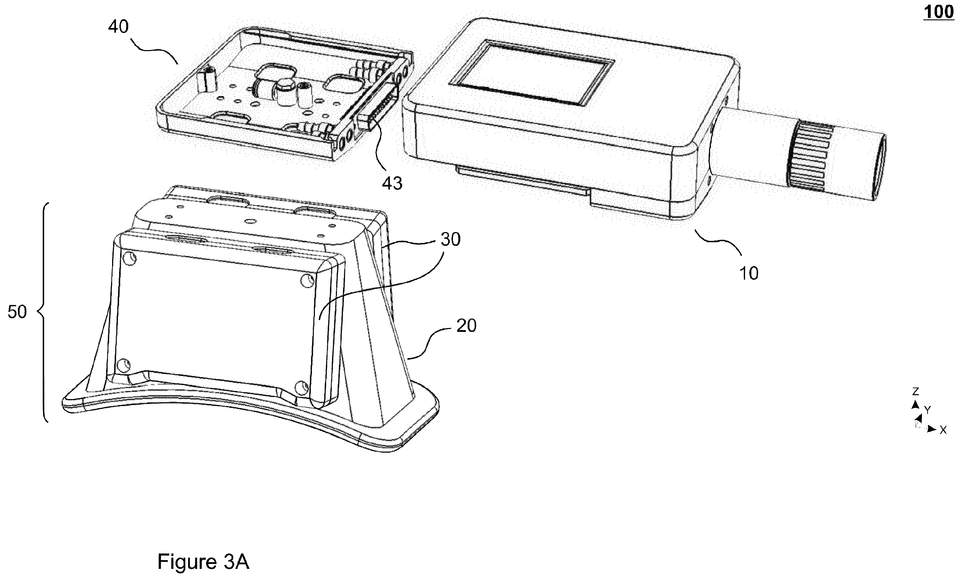

The assembly 100 further comprises an adapter 40 interposing the treatment head 50 and the handle 10. The adapter 40 mediates electric coupling between the control terminal 14 of the handle 10 and the cooling units 30 by means of appropriate electric circuitry. The adapter 40 is further configured to comprise at least one plug-in connector, such a standard D25 (male) connector 43 (Fig. 3A), compatible to a corresponding female connector part arranged within the handle 10, as described further. To those skilled in the art it is clear that any other appropriate connector may be utilized.

In one preferred embodiment the assembly 100 is implemented such that upon disengaging the treatment head 50 from the handle 10 the adapter 40 remains mounted onto the treatment head 50 and, in particular, onto the vacuum cup applicator 20 (as shown in Fig. 1). In another preferred embodiment the assembly 100 is configured such that upon disengaging of the aforementioned components the adapter 40 remains connected to the handle 10.

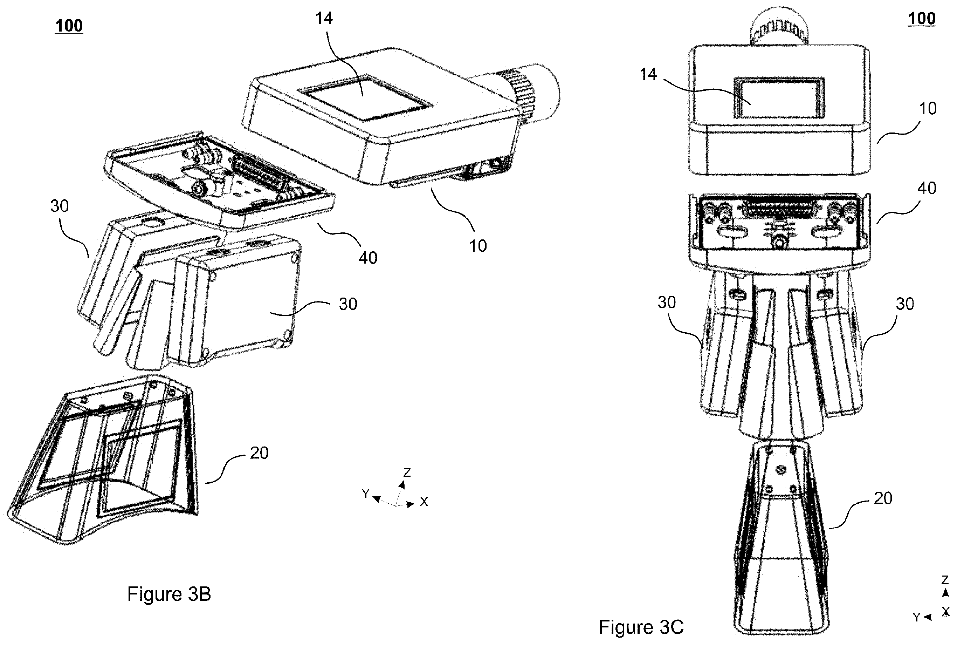

Figs. 3B and 3C provide exploded view of the assembly 100 and the main components thereof. The assembly 100 thus comprises the vacuum cup applicator 20 disposed between two cooling units 30. The assembly 100 may be embodied to comprise at least one cooling unit 30 at either size of the elongated vacuum cup applicator 20. General configuration of the cooling units 30 is such to provide a most complete coverage of skin and tissue enclosed within the treated area by means of vacuum suction. Detailed exploded view of a single cooling unit 30 with regards to the vacuum cup applicator 20 is given at Fig. 1.

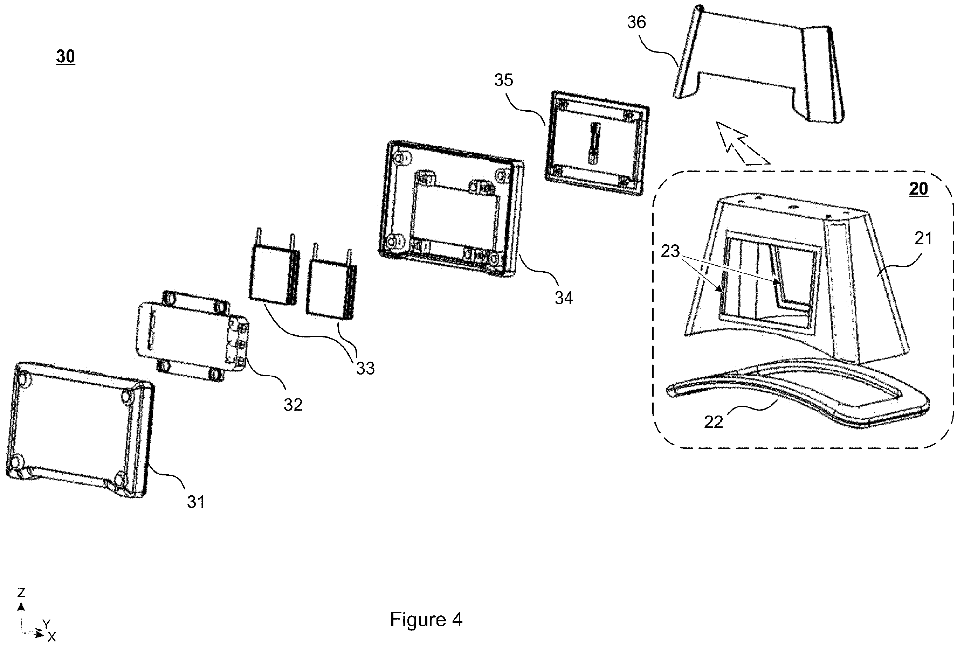

The vacuum cup applicator 20 is advantageously provided as an elongated, cup-shaped body applicator configured to impart vacuum force onto the skin of a patient. In preferred embodiment the vacuum cup applicator 20 consists of a cup-shaped frame 21 and a corresponding edge 22 components (Fig. 4, dashed box). While the frame 21 is advantageously produced from a relatively hard material, which is capable of preserving constant shape while vacuum is drawn in the interior cavity, the edge 22 is preferably produced from a softer material in order to achieve an enhanced fitting onto the treated area. In one exemplary embodiment the frame 21 may be manufactured from plastic and the edge 22 - from rubber, such as silicone polymer. In one preferred embodiment the silicone polymer edge 22 is detachable and replaceable; configuration and geometrical parameters thereof may be adjusted accordingly. Thus, the vacuum cup applicator may be realized as a kit, comprising a sufficiently rigid frame 21 and a number of edge components 22, each edge component adjusted to fit a predetermined area to be treated, e.g. arms, thighs, abdomen and so forth. Moreover, the edge components 22 may be adjusted to fit individuals of different body size or even tailored for certain patients.

The vacuum cup applicator 20 or the frame component 21 further comprises a vacuum intake port/aperture arranged in a surface thereof adjacent to the handle 10/the adapter 40.

In some other embodiment the vacuum cup 20 may be realized as a solid structure, having a stiff frame 21 permanently fixed to a softer edge 22. In some alternative embodiments the components 21, 22 may be produced from the same material.

The vacuum cup applicator 20 further incorporates fitting appliances for the adapter 40. In some embodiment the vacuum cup applicator 20 is further configured to comprise one or more light sources (not shown), preferably configured as low-level laser LEDs, and appropriate electric circuits. Preferably, the vacuum cup applicator comprises 4-10 light sources, arranged within the bottom (a portion facing an interior cavity) of the vacuum cup applicator; such an arrangement ensures optimal protection rates from excessive cooling. Light sources may be arranged either on the surface (internal or external) of the vacuum cup applicator 20 or, alternatively, embedded into the plastic material said vacuum cup 20 is made from. In another embodiment placement of the light sources is targeted to the adapter 40; in still another embodiment the light sources are allocated to the handle 10.

In preferred embodiment the vacuum cup applicator 20 comprises two apertures 23 arranged in the elongated side faces of the frame 21. By means of said apertures the vacuum cup 20 is coupled to each of the cooling units 30 in a manner shown in Figs. 3B and 3C.

In preferred embodiment the assembly 100 comprises two cooling units 30. Each of said cooling units 30 is preferably configured as a thermoelectric cooling unit and preferably exploits the principles of Peltier effect based cooling. With reference to Fig. 4, each cooling unit 30 may include a casing, comprising an outer cover 31 and an inner cover 34, at least one thermoelectric cooler (TEC) 33, a heat transfer element 32, a first cold plate 35 and a second cold plate 36. When assembling the treatment head 50, the first cold plate 35 is disposed to fit into the aperture 23 arranged in each side face of the vacuum cup applicator 20, whereas the second cold plate 36 allocates within an interior of said vacuum cup applicator 20. The second cold plate 36 by size and shape thereof is thus adjusted to conform to an interior cavity of the vacuum cup applicator 20.

Each cold plate 35, 36 is preferably made of thermally conductive material, such as aluminum or copper. Cold plate may be manufactured from any other material, provided said material is sufficiently stiff and thermally conductive. The cold plates resist deformation and maintain a consistent mechanical and thermal interface between the TECs and the treated area. In particular, the rigidness requirement concerns the cold plate 36, since it undergoes vacuum impact during treatments. Concave profile of the cold plate 36 ensures heat extraction of the most of the treated area.

In the embodiment shown in Fig. 4 each cooling unit 30 comprises preferably two thermoelectric cooler (TEC) elements 33. Each TEC element 33 is preferably realized as a Peltier element capable of transferring heat to either side of itself. Each TEC element 33, configured hereby as a Peltier cooler, is arranged to transfer heat from a "cold" interface, being thermally coupled to the cold plates 35 and 36 to a "hot" interface, being thermally coupled to the heat transfer or dissipation element 32. The cooling unit 30 may further comprise at least one electric circuit (not shown) for connecting the TEC elements 33 out of the module 30. Mentioned electric circuit, in addition to required wiring and appropriate connectors may further comprise a number of power controllers for TEC elements 33, an aforementioned light source or light sources, such as a laser LEDs, wiring and control electronics for said light source(s), a pressure sensor indicating vacuum level relative to ambient, a contact sensor indicating the contact of the said apparatus to skin, a microcontroller or similar control terminal, an indicator light, a heat sensor for a hot side of a TEC, a heat sensor for a cold side of a TEC, a power usage sensor and/or a sensor indicating that the a TEC module is connected.

In one preferred embodiment each cooling unit 30 comprises the heat transfer element 32, implemented as a so called heat pipe element. Flat heat pipes of a so called vapour chamber type are preferably utilized. A thin planar heat pipe is realized as a hermetically sealed hollow shell or vessel provided with a closed-loop capillary recirculation system (capillary wick structure), in which a working fluid is circulating. The wick structure lines the inner surface of the heat pipe shell and is saturated with the working fluid. The wick provides the structure to develop the capillary action for the liquid returning from the condenser (heat output; the outer cover 31) to the evaporator (heat input; the TECs 33). At a "hot" side of a heat pipe being in contact with the TECs 33 the working liquid turns into a vapor as a result of heat absorption. The vapor spreads along the heat pipe towards a "cold" side being in contact with ambient (represented hereby by the outer cover 31) using pressure generated by the temperature difference and condenses back into a liquid while releasing the latent heat, which is rejected to ambient or, alternatively, to an external heat sink. The liquid then returns back to the "hot" interface through capillary action and the cycle repeats. The shell of the heat pipe element 32 may be manufactured from a variety of materials, such as aluminium, copper, titanium, stainless steel or any other appropriate material.

In the preferred embodiment the working liquid is acetone (operating temperature range -48 to 125 °C), however, any other working fluid with suitable operating temperature range, such as water (1 to 325 °C), methanol (-75 to 120 °C), propylene (-150 to 60 °C) or ammonia (-75 to 125 °C), for example, is not excluded.

In some supplementary embodiments each cooling unit 30 may still comprise, additionally or alternatively to the heat pipe element 32, at least one heat exchanger containing a number of water circulation channels.

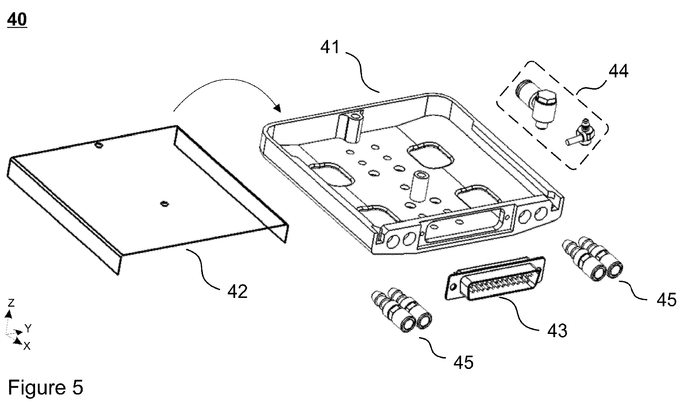

Fig. 5 is an exploded view of the adapter 40. By means of the adapter 40 the treatment head 50, thus comprising the vacuum cup applicator 20 and the cooling units 30, is mechanically and/or functionally connected to the handle 10. By mechanical connection we refer to physical association between separate elements; whereas by functional connection we refer to association between physically integrated or non-integrated elements mediated by electrical signals. The embodiment shown in Fig. 5 comprises a component 41 constituting the adapter's body and connecting the adapter 40 to the vacuum cup applicator, said component 41 is advantageously covered by a covering- or protection plate 42. The adapter 40 shown in Fig. 5 further comprises the connector 43, two pairs of fittings 45 for mediating electrical and/or mechanical connection to the handle 10 and a vacuum gauge, indicated in Fig. 5 by reference numeral 44 and realized as an angle connector. A casing, thus formed by the components 41 and 42 may further comprise a variety of light sources, such as aforementioned laser LEDs, sensors and appropriate electric circuits.

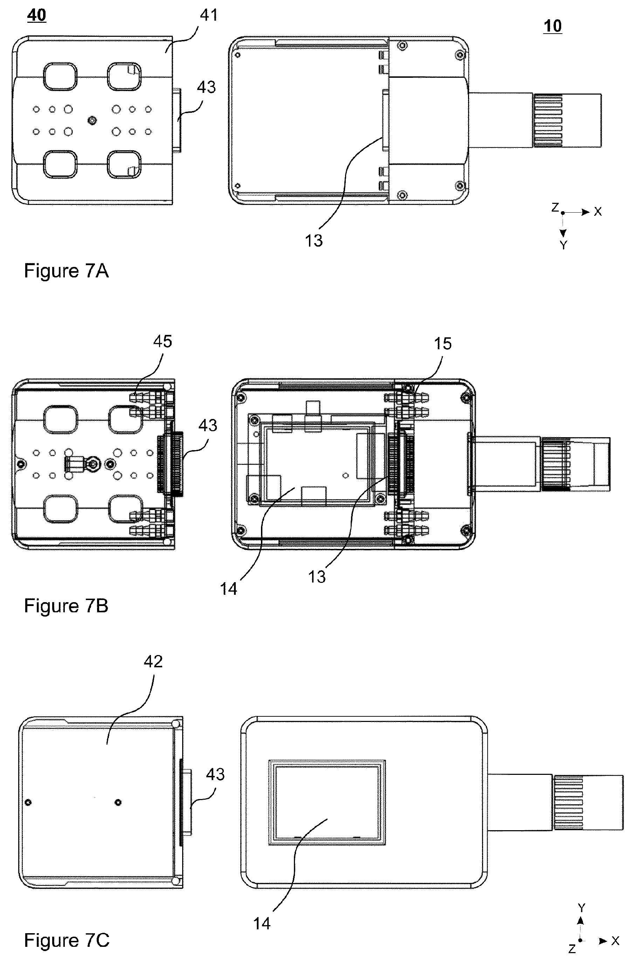

Figs. 7A-7C show disposition of the adapter 40 with regards to the handle 10. Fig. 7A shows a bottom view of the adapter 40, i.e. a treatment head 50 connection interface thereof. The adapter 40 is mechanically connected to the handle 10 by means of a standard plug-in connector indicated by reference numerals 43 (male connector part of D25 type, adapter 40) and 13 (female connector part of D25 type, handle 10) and/or fittings 45 and 15.

Configuration of the adapter 40 and its contacts with the treatment head 50 and the handle 10 is flexible. Provision of the assembly 100 according to the one preferred embodiment is such that connection between the adapter 40 and the treatment head 50 is fixed. The adapter 40 thus remains mounted onto the vacuum cup applicator 20 upon disengaging the treatment head 50 from the handle 10 (as shown in Fig. 1). In another preferred embodiment the assembly 100 may be realized to have a releasable connection between the treatment head 50 and the adapter 40. The adapter would thus remain connected to the handle 10 while the treatment head 50 (comprising the vacuum cup application 20 and the cooling units 30) is released from the handle 10 and from the adapter 40, correspondingly. In both embodiments the adapter is preferably realized as a separate, replaceable component.

The aforesaid connection modes between: a. the adapter 40 (coupled to the handle 10) and the treatment head 50; and b. the adapter 40 (coupled to the treatment head 50) and the handle 10, may be realized by various means. In some embodiments the connection may be simply electro-mechanical, such as a plug-in connection. In some other embodiments the connection may be pneumatic, preferably vacuum relative to ambient. In some embodiments the connection may be hydraulically actuated. In some preferred embodiments the connection can be realized by means of magnetic coupling. In another preferred embodiments the mentioned connection can be realized as a combination of magnetic force and negative pressure (above mentioned vacuum). Since the assembly 100 in operation is connectable to the vacuum pump via the handle 10, negative pressure can be utilized as a type of "locking". Turning off vacuum causes suction release, therefore the treatment head 50 can be easily detached from the handle 10. By complementing said negative pressure based coupling by magnetic coupling it is ensured that the treatment head 50 would remain attached to the handle 10 also when vacuum is turned off.

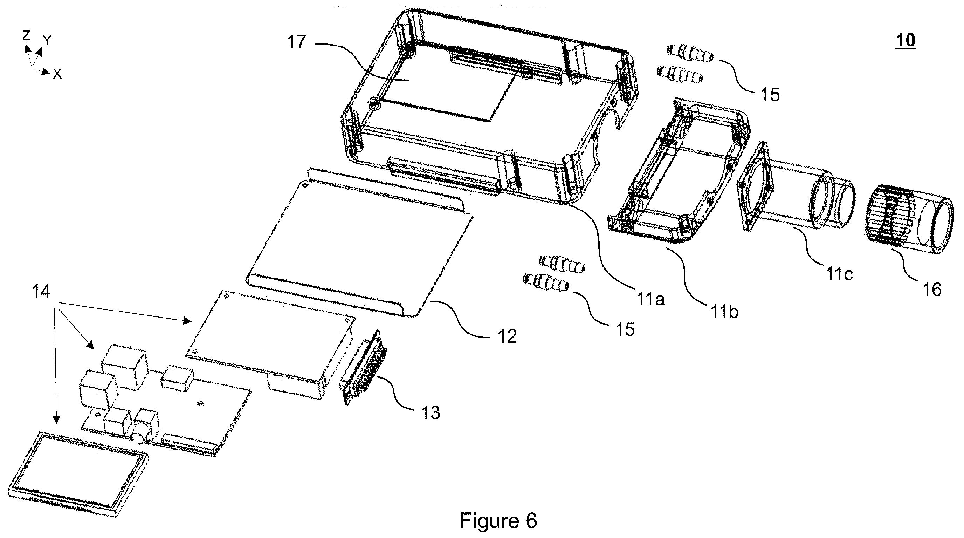

Referring back to Fig. 6, the handle 10 comprises a casing consisting of at least components 11 a, 11b, 11c and a protection plate 12. The component 11a constitutes a top cover, the component 11b and the protection plate 12 - a bottom cover and the component 11c provides a sheath for a fluidic line. A fluidic line connector 16 constitutes a manual adjusting control and encompasses the sheath component 11c. The handle 10 comprises a set of fittings 15 complementary to the fittings 45 of the adapter 40. In the top cover 11a of the casing an aperture 17 is advantageously arranged for hosting at least one control terminal 14 equipped with the user interface. The user interface is realized as a graphical user interface in the form of a display screen, preferably a touchscreen. User interface may also comprise at least one audio input-output device and associated circuitry. User interface may further comprise a number of haptic (tactile) feedback component(s), such as actuators inducing vibration. The control terminal 14 is further provided with one or more processing devices containing a processing circuitry capable of interpreting and executing instructions input via the user interface, said processing devices being realized as microprocessors, microcontrollers, digital signal processors, programmable logic chips etc. In some embodiments the processing device is realized as a miniature, credit card-sized computer, such as a single-board Raspberry computer. The control terminal 14 is configured to acquire a direct electrical communication with each of the cooling units 30 when the assembly 100 is in operation.

Via the user interface the control terminal 14 allows adjusting settings for vacuum power, temperature and treatment time individually for each handle 10. As the assembly 100, and, in particular, the handle 10 may be considered as a peripheral device with regards to the treatment unit/the apparatus for cosmetic treatment, the control terminal 14 provided within each handle 10 is further configured to communicate with the central control terminal/processor provided within the treatment unit/the apparatus for cosmetic treatment and/or with any other remote computer/server; and to mediate synchronization of operation settings between a number of said peripheral devices. Communication may be wired and/or wireless; thereby the control terminal 14 may further comprise a wireless transceiver, including, but not limited to a short-range IR transceiver, a low-power RF transceiver and a Bluetooth™ transceiver.

Additionally, each handle 10 may be configured to comprise an ON/OFF switch for manual triggering the power switch operation. The switch action is typically controlled via the aforementioned central control terminal/processor provided within the treatment unit/the apparatus for cosmetic treatment.

Configuration of the assembly 100 is preferably such that a single handle 10 may connect to a number of treatment heads 50 of variable size and shape. In this regard, the treatment head 50 and, in particular, the vacuum cup applicator 20, may be embodied to fit different body areas, including arms, thighs and calves, higher and lower abdomen, love handles, buttocks, back and so forth. The treatment head 50 and/or the whole assembly 100 may be further size-adjusted to fit smaller target areas, such as chin, for example. Configuration of the assembly 100 relies on a so called hot swapping principle and allows changing and/or replacing the treatment head 50 without switching power off the handle 10 and/or an actual apparatus for cosmetic treatment.

In the preferred embodiment the assembly 100 is further configured to comprise a thermographic camera, configured to comprise at least one thermal sensor. Said sensor may be an infrared sensor; however, any other appropriate operation principle is not excluded. In one preferred embodiment the thermographic camera and/or thermal sensor(s) may be integrated into the vacuum cup applicator 20. In some other embodiment the thermographic camera and/or thermal sensor(s) may be integrated into the adapter 40. The thermographic camera and/or each thermal sensor are configured to measure temperature of tissue drawn inside the vacuum cup upon application of vacuum suction onto the treated area. Incorporation of the thermographic camera or the thermal sensor into the assembly 100 allows real-time monitoring of tissue temperatures and fluctuations thereof with regards to each patient. Based on readings (a thermal map) provided by the thermographic camera temperature settings for each assembly 100 may be adjusted individually either manually or automatically. In a latter case a control terminal 14 may be preprogrammed to comprise temperature threshold data. Mentioned thermal map may be visualized by means of a user interface provided within the treatment unit/the apparatus for cosmetic treatment and/or communicated to another electronic device, including, but not limited to PC, portable or tablet computer, mobile phone, smart phone, PDA and the like.

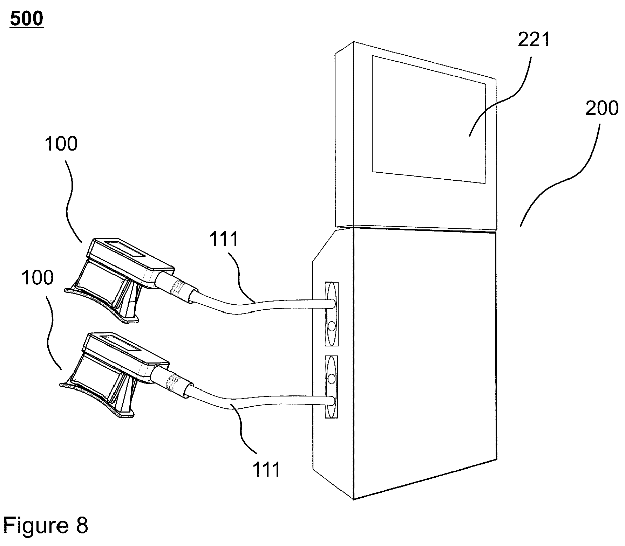

Fig. 8 shows the apparatus 500 for non-invasive cosmetic treatment and comprising a treatment unit 200 and a number of the handpiece assemblies 100, implemented in accordance with any embodiment disclosed above. For clarity purposes the components 200 and 100 (Fig. 8) are not necessarily in scale. The treatment unit 200 preferably comprises a central control terminal/processor system configured to regulate fluidic medium and/or electric signal communication between said treatment unit and/or accessory appliances thereof and each handpiece assembly 100. The treatment unit 200 further comprises a user interface 221, preferably a graphical user interface. The user interface comprises a display screen, preferably a touchscreen. Connection between the treatment unit 200 and each assembly 100 is realized by a number of fluidic- and/or signal communication lines 111, preferably gathered within a common sheath. Said lines 111 include, but are not limited to coolant input and output lines, one or more vacuum lines and one or more electric signal communication lines. In one preferred embodiment connection between each line 111 and the treatment unit 200 is realized based on a hot swapping principle. Lines 111 can thus be removed, replaced and plugged-in again to any connection port localized within the treatment unit 200 without shutting down said apparatus 500.

In some further additional or alternative embodiments also connection between any of the lines 111 and the handle 10 of each assembly 100 may rely onto aforesaid hot swapping principle. Thus, the handle 10 of each assembly 100, connected to the fluidic- and/or signal communication line 111 by means of the exemplary fluidic line connector 16, can be unrestrictedly detached, replaced and attached again to any fluidic- and/or signal communication line 111 provided with the apparatus 500 without shutting down said apparatus. It is worth mentioning that the connector 16 may be any type of connector suitable for coupling the handle 10 to the line 111.

In some embodiments the apparatus 500 may be configured such, that detaching of an individual handpiece assembly 100 from a corresponding fluidic- and/or signal communication line 111 or detaching of said corresponding fluidic- and/or signal communication line 111, coupled to the handpiece assembly 100, from the treatment unit 200 will cause the control system of the apparatus 500 to acquire a stand-by mode. In stand-by mode such operational functions mediated by the apparatus 500 and/or the treatment unit 200 thereof, as coolant circulation, electric current supply and vacuum supply, are interrupted with regard to each individual handpiece assembly 100. Thus the apparatus 500 may be advantageously configured to interrupt the at least coolant circulation, electric current supply and vacuum supply operational functions with regard to each individual handpiece assembly 100 upon detaching of said handpiece assembly 100 from the corresponding fluidic- and/or signal communication line 111 or upon detaching of said corresponding fluidic- and/or signal communication line 111, coupled to the handpiece assembly 100, from the treatment unit 200; while operation of the other (connected) lines 111 and/or assemblies 100 will be still be maintained.

The apparatus 500 may still be configured to interrupt aforesaid operational functions with regard to a total number of handpiece assemblies 100 connectable to the treatment unit 200, even if only one particular assembly 100 is actually disconnected from said treatment unit 200. The apparatus 500 may be further configured to switch between the aforesaid modes of interruption.

Reactivation of the control system in all aforesaid embodiments and resumption of the operational functions, accordingly, is initiated upon restoration of physical connection between the handpiece assembly 100 and the treatment unit 200 via the line 111. Thus, operational functions are restored by plugging the fluidic- and or signal communication line 111 back to the treatment unit 200 and/or by coupling the handpiece assembly 100 to the corresponding line 111 by means of the appropriate connector provided on the handle 10 of said assembly.

Interruption and restoration of aforesaid operational functions are mediated by at least a number of detectors, sensors and the like, provided within each assembly 100 (the handle 10) and the treatment unit 200, and being in communication with the control terminal 14 provided within the assembly 100 (the handle 10) and the central control terminal/processor provided within the treatment unit 200.

It is clear to a person skilled in the art that with the advancement of technology the basic ideas of the present invention may be implemented in various ways without diverging from the fulcnim of the present invention. The invention and its embodiments are thus not limited to the examples described above; instead they may generally vary within the scope of the appended claims.

## Claims

1. A handpiece assembly (100) for an apparatus used in non-invasive cosmetic treatment, said handpiece assembly comprising:
- an electronically controlled handle (10) provided with a control terminal (14) equipped with a user interface, said handle (10) being connectable to the treatment unit of the apparatus for cosmetic treatment by a number of fluidic lines and signal communication lines,
- a treatment head (50) comprising a cup-shaped body applicator (20) and at least two cooling units (30), wherein the cup-shaped body applicator (20) is disposed between said at least two cooling units (30), and
- an adapter (40) implemented as a separate, replaceable component, configured to releasably connect the electronically controlled handle (10) and the treatment head (50), while said handle (10) and the related apparatus for cosmetic treatment being maintained in a functional switch-on state.

2. The handpiece assembly (100) of claim 1, wherein the adapter (40) is configured to mediate detachment of the treatment head (50) from and reconnection thereof to the electronically controlled handle (10), while said handle (10) and the related apparatus for cosmetic treatment being maintained in a functional switch-on state.

3. The handpiece assembly (100) of claim 1, wherein the adapter (40) is configured to communicate electrical signals between each cooling unit (30) and the handle (10).

4. The handpiece assembly (100) of claim 1, wherein the adapter (40) mediated connection between the handle (10) and the treatment head (50) is electro-mechanical and implemented by means of at least one plug-in connector (13, 43).

5. The handpiece assembly (100) of claim 1, wherein connection between the handle (10) and the treatment head (50) is magnetic and/or mediated by negative pressure.

6. The handpiece assembly (100) of claim 1, wherein the cup-shaped body applicator (20) is a vacuum cup applicator and comprises a cup-shaped frame element (21) configured to preserve constant shape while vacuum is drawn into the interior cavity thereof and an edge element (22) configured to provide tight fitting onto the treated area.

7. The handpiece assembly (100) of claim 1, wherein each cooling unit (30) is configured as a thermoelectric cooling unit and comprises at least one thermoelectric cooler element (33) and at least one heat transfer element (32) configured as a flat planar heat pipe consisting of a hermetically sealed vessel provided with a closed-loop capillary recirculation system with a working fluid and having a hot side in contact with said at least one thermoelectric cooler element (33) and a cold side in contact with an ambient.

8. The handpiece assembly (100) of claim 1, further comprising at least one light source in the form of a low-level laser LED, said light source being incorporated into the cup-shaped body applicator (20) or into the adapter (40).

9. The handpiece assembly (100) of claim 1, further comprising at least one thermal camera sensor configured to monitor temperature of subcutaneous tissue within the treated area, said camera sensor being incorporated into the cup-shaped body applicator (20) or into the adapter (40).

10. The handpiece assembly (100) of claim 9, configured, by means of the at least one thermal camera sensor, to monitor temperature fluctuations of subcutaneous tissue within the treated area in real time, and to further adjust, via the control terminal (14) equipped with a user interface, the treatment temperature settings based on readings provided by said thermal camera sensor and the temperature threshold data comprised in the control terminal (14).

11. The handpiece assembly (100) of any of claims 1-10 suitable for use with an apparatus for non-invasive fat reduction by means of cold-induced lipolysis of fat cells.

12. An apparatus (500) for non-invasive cosmetic treatment comprising a treatment unit (200) and a number of the handpiece assemblies (100) implemented according to any of claims 1-9, wherein each handpiece assembly (100) is releasably connected to the treatment unit (200) by a number of fluidic and/or signal communication lines (111) including a coolant fluidic line, a vacuum line and an electrical signal communication line, while the apparatus (500) being maintained in a functional switch-on state.

13. The apparatus (500) of claim 12, wherein each individual fluidic- and signal communication line (111) coupled to the handpiece assembly (100) is configured detachable, replaceable and reconnectable with regard to any connection port localized within the treatment unit (200), and each individual handpiece assembly (100) is further configured detachable, replaceable and reconnectable with regard to any fluidic- and signal communication line (111) coupled to the treatment unit (200), while the apparatus (500) being maintained in a functional switch-on state.

14. The apparatus (500) of claims 12 and 13, in which control over operational functions for each individual fluidic- and communication line is implemented in an independent manner such that disruption and restoration of an at least coolant circulation, electric current supply and vacuum supply operational functions with regard to each individual fluidic- and signal communication line upon detachment and reconnection thereof, accordingly, to the treatment unit (200) and to any individual handpiece assembly (100), is independent on disruption and restoration of said operational functions with regard to any other individual line (111) comprised in the apparatus (500).

15. The apparatus (500) of any of claims 12-14, being an apparatus for non-invasive fat reduction by means of cold-induced lipolysis of fat cells.
